# EUROPEAN PATENT APPLICATION

(11) **EP 1 892 218 A1**
(43) Date of publication of application: **27.02.2008**
(21) Application number: 06747119.3
(22) Date of filing: 02.06.2006
(51) Int. Cl.: C01G 9/00

(54) **DEGRADABLE ZINC OXIDE POWDER AND PROCESS FOR PRODUCTION THEREOF**

(30) Priority: 02.06.2005 JP 2005163145; 02.06.2005 JP 2005163146
(71) Applicant: Shiseido Company, Limited, Chuo-ku Tokyo 104-8010 (JP)
(72) Inventor: SHIO, Shoichiro, c/o SHISEIDO RESEARCH CENTER, Yokohama-shi, Kanagawa 2248558 (JP); ISHIKAWA, Yuko, c/o SHISEIDO RESEARCH CENTER, Yokohama-shi, Kanagawa 2248558 (JP); SUDA, Yukimitsu, c/o SHISEIDO RESEARCH CENTER, Yokohama-shi, Kanagawa 2248558 (JP); WADA, Masayoshi, c/o SHISEIDO RESEARCH CENTER, Yokohama-shi, Kanagawa 2248558 (JP)
(74) Representative: Henkel, Feiler & Hänzel
(86) International application number: PCT/JP2006/311089
(87) International publication number: WO 2006/129793

(57) **Abstract**

The method for producing a disintegratable zinc oxide powder of the present invention is **characterized by** comprising: neutralizing an aqueous solution containing a water-soluble zinc salt and a carboxylic acid or an aqueous solution containing a water-soluble zinc carboxylate with an alkali carbonate to produce precipitate; and firing the precipitate to obtain zinc oxide powder. As a water-soluble zinc salt, zinc chloride or zinc acetate is preferably used. As an acid, acetic acid is preferably used. As an alkali carbonate, sodium carbonate is preferably used. The disintegratable zinc oxide powder has the morphology in which the primary particles of zinc oxide are aggregated to form secondary particles, and these secondary particles are further aggregated to form the zinc oxide powder. The powder can achieve excellent UV protection capability, excellent transparency, and excellent feeling in use through its disintegration. Also, it has good usability and good handling properties.

## Description

### RELATED APPLICATIONS

This application claims the priority of Japanese Patent Application No. 2005-163145 and Japanese Patent Application No. 2005-163146 filed on June 2, 2005, which are incorporated herein by reference.

### FIELD OF THE INVENTION

The present invention relates to zinc oxide powder, and in particular, relates to disintegratable zinc oxide powder that has good usability and good handling properties and that can achieve excellent UV protection capability, excellent transparency, excellent feeling in use, etc. through its disintegration and also relates to the method of producing the same.

### BACKGROUND OF THE INVENTION

In the past, the achievement of transparency was pursued for the zinc oxide that is used as a UV protection agent in cosmetics. As a result, ultrafine particle powder with an average particle size of 100 nm or less has been predominantly used.

The production of zinc oxide is mainly classified into a dry method and a wet method. As for dry methods, these include the French process (indirect method) and American process (direct method); in these processes, zinc oxide is produced by the oxidation of zinc vapor by air. The dry method is the main method for the production of ordinary zinc oxide. However, for the production of UV-shielding ultrafine particulate zinc with a particle size of 100 nm or less, a wet process has been mainly used.

The wet process is a method in which an aqueous solution of a zinc salt is neutralized with an alkaline chemical, and the formed precipitate of zinc salt is washed with water, dried, and fired to obtain zinc oxide. However, this method has an issue in that the formed fine particles with the size of 100 nm or less aggregate during firing and the particle size increases, resulting in the loss of the transparency and UV-shielding properties. In addition, it is difficult to freely control the size of primary particles and the state of aggregation. Even when fine particles of zinc oxide are obtained, the usability and handling properties are poor because of fine particles, and it is difficult to use them as they are in cosmetics etc.

Therefore, various forms of zinc oxide have been proposed. For example, in Japanese Unexamined Patent Publication H01-230431 and Japanese Unexamined Patent Publication H06-115937, flaky zinc oxide powder is described. However, if the flaky powder is thin, the powder strength is not sufficient during the production of cosmetics etc. If the thickness is increased in order to increase the strength, the powder particle size becomes large. As a result, the transparency becomes low because of visible light scattering, and a problem arises in actual usage.

In Japanese Unexamined Patent Publication H05-139738, fine particles of hollow spherical zinc oxide are described. However, it is difficult to form fine particles because this hollow zinc oxide is prepared in a gas phase. In order to prepare fine particles of zinc oxide with UV protection capability, the loading concentration should be small and it is not suitable for mass production.

Although various other methods have been tried, there are few satisfactory methods.

### DISCLOSURE OF THE INVENTION

### PROBLEM TO BE SOLVED BY THE INVENTION

The present invention was made in view of the problem described in the background art, and the objects of the invention are to provide zinc oxide powder that is good in usability and handling properties and excellent in the UV protection capability, transparency, and feeling in use and to provide its production method. In addition, the present invention provides the cosmetics containing the same.

### MEANS TO SOLVE THE PROBLEM

The present inventors have diligently studied to solve the above-mentioned problem. As a result, it was found that it was possible, by a specific production method, to obtain zinc oxide powder, wherein fine primary particles aggregated by their loose connection with each other to form rod-like, plate-like, block-like, or tubular secondary particles, and the secondary particles further aggregated to form the zinc oxide powder. It was discovered that this zinc oxide powder could be easily disintegrated to primary particles by grinding, friction, etc.; as a result, excellent UV protection capability (especially in UV-A protection) and visible-light transmission (transparency) could be achieved, thus leading to completion of the present invention.

The method for producing a disintegratable zinc oxide powder of the present invention is characterized by comprising: neutralizing an aqueous solution containing a water-soluble zinc salt and a carboxylic acid or an aqueous solution containing a water-soluble zinc carboxylate with an alkali carbonate to produce precipitate; and firing the precipitate to obtain the zinc oxide powder, wherein zinc oxide primary particles are aggregated by their loose connection with each other to form secondary particles, and the secondary particles are further aggregated to form the zinc oxide powder.

In the method of the present invention, it is preferable that the water-soluble zinc salt is zinc chloride.

It is also preferable that the carboxylic acid is acetic acid.

It is also preferable that the water-soluble zinc carboxylate is zinc acetate.

It is also preferable that the alkali carbonate is sodium carbonate.

It is also preferable that in the aqueous solution containing the water-soluble zinc salt and the carboxylic acid or in the aqueous solution containing the water-soluble zinc carboxylate, the amount of carboxyl group is 0.1 to 10 times in moles relative to the amount of zinc, and more preferably 0.5 to 5 times in moles.

It is also preferable that the alkali carbonate is 0.1 to 10 mol/L aqueous solution thereof per 100 mL of the aqueous solution containing the water-soluble zinc salt and the carboxylic acid or per 100 mL of the aqueous solution containing the water-soluble zinc carboxylate. In addition, it is preferable that the alkali carbonate aqueous solution is dropwise added, for neutralization, at a rate of 0.1 to 10 mL/min.

The secondary particles can be tubular. In this case, an aqueous solution containing zinc chloride and acetic acid or an aqueous solution containing zinc acetate is neutralized with sodium carbonate to produce precipitate, and the precipitate is fired to obtain the zinc oxide powder, wherein zinc oxide primary particles are aggregated to form tubular secondary particles, and the tubular secondary particles are further aggregated to form the zinc oxide powder. It is preferable that the sodium carbonate is 0.1 to 2 mol/L aqueous solution thereof per 100 mL of the aqueous solution containing zinc chloride and acetic acid or per 100 mL of the aqueous solution containing zinc acetate. It is also preferable that, in the aqueous solution containing zinc chloride and acetic acid or in the aqueous solution containing zinc acetate, the amount of acetic acid is 1 to 10 times in moles relative to the amount of zinc. It is also preferable that the sodium carbonate aqueous solution is dropwise added, for neutralization, at a rate of 0.5 to 5 mL/min per 100 mL of the aqueous solution containing zinc chloride and acetic acid or the aqueous solution containing zinc acetate.

One example of the disintegratable zinc oxide powders of the present invention is characterized by the zinc oxide powder, wherein zinc oxide primary particles are aggregated to form secondary particles, and the secondary particles are further aggregated to form the zinc oxide powder, which is obtained by any of the above-described methods. In the present powder, it is preferable that the average particle size of the primary particles is 0.02 to 0.1 µm, the average particle size of the secondary particles is 0.5 to 10 µm, and the average particle size of aggregates of the secondary particles is 1 to 100 µm.

In addition, one example of the disintegratable zinc oxide powders of the present invention is characterized by the zinc oxide powder, wherein zinc oxide primary particles are aggregated to form tubular secondary particles, and the tubular secondary particles are further aggregated to form the zinc oxide powder. The present powder can be obtained by the above-described method. The average major axis of the tube can be 0.5 to 10 µm. The ratio of major axis/minor axis of the tube can be one or higher.

The cosmetic of the present invention is characterized by comprising any of the above-described disintegratable zinc oxide powder and/or its disintegrated material.

The particle size of zinc oxide powder of the present invention is large; thus there is no dispersal of fine particle powder, and the aggregation ofthe power hardly takes place even in a base material to which the powder is blended. Thus, it is excellent in usability and in handling properties.

The zinc oxide powder of the present invention disintegrates easily to primary particles by grinding, friction, etc. As a result, the excellent UV protection capability (especially in UV-A protection) and the visible-light transmission (transparency) can be achieved.

In addition, the disintegratable zinc oxide powder and/or its disintegrated material of the present invention improve the slip property of cosmetics.

### BRIEF DESCRIPTION OF THE DRAWINGS

Fig. is an SEM micrograph of the disintegratable zinc oxide powder (Test Example 1) in accordance with one example of the present invention.
Fig. 2 is a TEM micrograph of the disintegratable zinc oxide powder (Test Example 1) in accordance with one example of the present invention.
Fig. 3 shows spectral transmittance curves for the disintegratable zinc oxide powder (Test Example 1) in accordance with one example of the present invention and for a commercial ultrafine particulate zinc oxide powder.
Fig. 4-1 is an SEM micrograph, before dispersion, of the disintegratable zinc oxide powder (Test Example 1) in accordance with one example of the present invention.
Fig. 4-2 is an SEM micrograph, after dispersion, of the disintegratable zinc oxide powder (Test Example 1) in accordance with one example of the present invention.
Fig. 5 is an SEM micrograph of zinc oxide powder (Test Example 2-5) obtained by using zinc chloride with phosphoric acid as an acid.
Fig. 6 is an SEM micrograph of zinc oxide powder (Test Example 2-1) obtained by using zinc chloride without an acid.
Fig. 7 is an SEM micrograph of zinc oxide powder (Test Example 2-2) obtained by using zinc chloride with propionic acid as an acid.
Fig. 8 is an SEM micrograph of the zinc oxide powder (Test Example 2-3) obtained by using zinc chloride with maleic anhydride as an acid.
Fig. 9 shows a relationship between the pH of the reaction solution and the dropping time when a sodium carbonate aqueous solution is dropwise added, for neutralization, to a zinc chloride aqueous solution in the presence of acetic acid (Test Example 1) and in the absence of acetic acid (Test Example 2-1).
Fig. 10 shows spectral transmittance curves for the powders obtained by using sodium carbonate (Test Example 1) or sodium hydroxide (Test Example 3) as an alkaline chemical.
Fig. 11 shows spectral transmittance curves for the powders obtained when the amount of used acetic acid was varied in Test Example 1.
Fig. 12 shows spectral transmittance curves for the powders obtained when the concentration of sodium carbonate aqueous solution was varied in Test Example 1.
Fig. 13 shows spectral transmittance curves for the powders obtained when the dropping rate of sodium carbonate aqueous solution was varied in Test Example 1.

### BEST MODE FOR CARRYING OUT THE INVENTION

The disintegratable zinc oxide powder of the present invention can be obtained by neutralizing an aqueous solution containing a water-soluble zinc salt and a carboxylic acid or an aqueous solution containing a water-soluble zinc carboxylate (hereinafter, these aqueous solutions may be referred to as "zinc aqueous solution") by the addition of an alkali carbonate and by washing with water, as necessary, the obtained precipitate, drying, and then firing.

As the water-soluble zinc salt, salts normally used in the liquid phase method can be used. Examples thereof include zinc chloride, zinc nitrate, zinc sulfate, and zinc acetate. From the standpoint of disintegratability and functionality, zinc chloride or zinc acetate is preferably used, and particularly zinc chloride is preferably used. When zinc acetate is used as a water-soluble zinc salt, the addition of carboxylic acid may be omitted.

The concentration of the water-soluble zinc salt in the zinc aqueous solution containing is normally 0.1 to 5 mol/L at the start of the reaction.

As the carboxylic acid used with the water-soluble zinc salt, water-soluble carboxylic acids can be used. Examples thereof include formic acid, acetic acid, propionic acid, oxalic acid, citric acid, tartaric acid, succinic acid, and maleic anhydride. From the standpoint of disintegratability and functionality, acetic acid is desirable.

The amount of the carboxyl group of the carboxylic acid, such as acetic acid, can be 0.1 to 10 times in moles relative to the amount of zinc, and 0.5 to 5 times in moles is preferable. If the amount of the carboxylic acid is too small, the disintegratability and functionality are not satisfactory. The use of excess amount achieves no significant improvement in the effects. Thus, it is not economical and an undesirable effect to disintegratability may be caused.

When a mineral acid such as hydrochloric acid is used instead of a carboxylic acid, disintegratable zinc oxide powder that is excellent in disintegratability and functionality cannot be obtained. Accordingly, it is considered that the carboxylate ion such as the acetate ion should be present with the zinc ion when the disintegratable zinc oxide powder of the present invention is produced by the neutralization with an alkali carbonate.

In order to achieve a complete dissolution state of a zinc aqueous solution, a mineral acid can be concomitantly used. Examples of mineral acids include hydrochloric acid, nitric acid, and sulfuric acid, and hydrochloric acid is preferably used.

As the alkali carbonate for neutralization, carbonate ion such as sodium carbonate or potassium carbonate is preferably used, and most preferably sodium carbonate is used.

When the above zinc aqueous solution is neutralized with an alkali carbonate, it is preferable that the alkali carbonate aqueous solution is continuously dropped into the zinc aqueous solution under stirring.

The concentration of the alkali carbonate aqueous solution is preferably 0.1 to 10 mol/L, and more preferably 0.3 to 5 mol/L.

The dropping rate of the alkali carbonate aqueous solution is preferably 0.1 to 10 mL/min per 100 mL of an aqueous solution of the zinc salt, and more preferably 1 to 5 mL/min.

If the concentration of an alkaline chemical or the dropping rate is too small, the reaction takes too long. As a result, the reaction is inefficient, and the disintegratability and functionality of the product may be unsatisfactory On the other hand, if the concentration of an alkaline chemical or the dropping rate is too large, the disintegratability and functionality of the product may also be unsatisfactory.

If another alkaline chemical such as sodium hydroxide is used instead of the above water-soluble carbonate, the desired disintegratability and functionality cannot be achieved. Accordingly, the neutralization with carbonate ion is preferable.

When an alkali carbonate aqueous solution is dropwise added to a zinc aqueous solution, a zinc salt starts to precipitate at a certain amount of the added alkali carbonate. When a solution containing zinc chloride and acetic acid is neutralized with a sodium carbonate aqueous solution, the pH of the reaction solution at the precipitation starting point is normally 5.5 to 6.5. Up to the precipitation starting point, the pH increases with an increase in the amount of the added alkali carbonate. However, the pH increase, with the progress of addition, is very mild after the start of precipitation because the alkali carbonate is consumed for the precipitation of the zinc salt. After the completion of precipitation, the pH increases rapidly. It is desirable that the addition is continued until the precipitation of the zinc salt is finished by the completion of the neutralization reaction. Normally, the addition until the pH of the reaction solution becomes about 8 to 8.5 is sufficient.

Thus, the neutralization reaction in the present invention progresses in two steps; the first step is up to the precipitation starting point, and the second step is after the start of precipitation.

It is preferable to carry out the reaction of the above zinc aqueous solution and the alkali carbonate aqueous solution at 40 °C or lower. If the reaction temperature exceeds 40°C, the satisfactory disintegratability and functionality may be insufficient. If the reaction temperature is too low, problems such as decreased efficiency in precipitation formation, precipitation of the raw materials, and freezing take place. Therefore, the reaction temperature should normally be 15 °C or higher, and preferably 25 °C or higher.

The obtained precipitate from the reaction is separated, as necessary, by known methods such as filtration and centrifugation, and the solid phase is washed with water and dried. At this stage, the aggregate of secondary particles is already formed; firing is conducted to conbert the aggregate to zinc oxide by decarboxylation.

The preferable firing temperature is 350 to 450 °C. If the firing temperature is too high, marked sintering takes place. As a result, the disintegratability is lowered, and the desired effect may not be achieved. On the other hand, if the firing temperature is too low, the generation of zinc oxide by firing is inefficient, and it is undesirable.

The firing time is suitably set so that it is sufficient for the formation of zinc oxide; it is normally 1 to 10 hours.

The disintegratable zinc oxide powder, which can be obtained as described above, of the present invention is of the following morphology. The primary particles of zinc oxide are loosely aggregated to form secondary particles, and these secondary particles are further aggregated to form the zinc oxide powder. Depending upon the types of used raw materials and the reaction conditions, the secondary particles take various shapes such as rod-like, plate-like, block-like, and tubular shapes. The aggregation patterns of the secondary particles are irregular, and no regular pattern is recognizable. The average particle size of the primary particles is normally about 0.02 to 0.1 µm, and the average particle size of the secondary particles is normally about 0.5 to 10 µm. The disintegratable zinc oxide powder of the present invention is an aggregate in which such secondary particles are connected to each other, and the average particle size is normally about 1 to 100 µm. The particle size was measured by electron microscope observation.

In the method of the present invention, the shape and connecting power of the secondary particles vary depending upon the types of used raw materials and reaction conditions. For example, when the disintegratable zinc oxide powder that is an aggregate of tubular secondary particles is desired, it is preferable to use the method in which an aqueous solution containing zinc chloride and acetic acid or an aqueous solution containing zinc acetate is neutralized with sodium carbonate, and the obtained precipitate is fired. This method is further explained in the following.

The disintegratable zinc oxide powder, the secondary particles of which is tubular, can be obtained by neutralizing an aqueous solution containing zinc chloride and acetic acid or an aqueous solution containing zinc acetate (hereinafter, these aqueous solutions may be referred to as "zinc aqueous solution"), at 40°C or less, by the addition of sodium carbonate, and by washing with water, as necessary, the obtained precipitate, drying, and then firing. Additional acetic acid can be used for the aqueous solution containing zinc acetate. It is preferable to use an aqueous solution containing zinc chloride and acetic acid rather than a zinc acetate aqueous solution because the UV protection capability and transparency tend to be better.

The concentration of zinc chloride or zinc acetate in the zinc aqueous solution is normally 0.1 to 5 mol/L at the start of reaction.

The amount of acetic acid used with zinc chloride is 1 to 10 times in moles relative to the amount of zinc, and it is preferably 1 to 5 times in moles. If the amount of acetic acid is too small, tubular particles cannot be formed. If the amount of acetic acid is too high, a loss of tubular shape tends to be observed.

Tubular secondary particles cannot be formed even when other carboxylic acids or mineral acids are used instead of acetic acid. Accordingly, it is considered that when the neutralization with sodium carbonate is conducted, the coexistence of acetate ion with zinc ion is necessary for the formation of tubular particles.

In order to achieve a complete dissolution state of the zinc aqueous solution, a mineral acid may be concomitantly used. Examples of mineral acids include hydrochloric acid, nitric acid, and sulfuric acid, and hydrochloric acid is preferably used.

When the zinc aqueous solution is neutralized with sodium carbonate, it is preferable that the sodium carbonate aqueous solution is continuously dropped into the zinc aqueous solution under stirring.

The concentration of the sodium carbonate aqueous solution is preferably 0.1 to 2 mol/L, and more preferably 0.2 to 1.5 mol/L.

The dropping rate of the sodium carbonate aqueous solution is preferably 0.5 to 5 mL/min per 100 mL of the zinc aqueous solution.

If the concentration of sodium carbonate or the dropping rate is too small, the reaction takes too long. As a result, the reaction is inefficient, and tubular particles may not be formed. On the other hand, if the sodium carbonate concentration or the dropping rate are too large, tubular particles may not also be formed and the functionality may be affected.

When other alkaline chemicals such as sodium hydroxide are used instead of sodium carbonate, tubular particles cannot be formed. Accordingly, it is considered that the neutralization with a carbonate ion is necessary.

It is preferable to carry out the reaction between the zinc aqueous solution and the sodium carbonate aqueous solution at 40 °C or lower. If the reaction temperature exceeds 40 °C, the formation of tubular particles, disintegratability, or functionality may not be satisfactory. If the reaction temperature is too low, problems such as decreased efficiency of precipitation formation, precipitation of the raw materials and freezing take place. Therefore, the reaction temperature should normally be 15 °C or higher, and preferably 25 °C or higher.

The obtained precipitate from the reaction is separated, as necessary, by known methods such as filtration and centrifugation, and the solid phase is washed with water and dried. At this stage, the aggregate of tubular secondary particles is already formed; firing is conducted to convert the aggregate to zinc oxide by decarboxylation.

The preferable firing temperature is 350 to 450 °C. If the firing temperature is too high, marked sintering takes place. As a result, disintegratability is lowered, and the desired effect may not be achieved. On the other hand, if the firing temperature is too low, the generation of zinc oxide by firing is inefficient, and it is not desirable.

The firing time is suitably set so that it is sufficient for the generation of zinc oxide, and it is normally 1 to 10 hours.

In the zinc oxide powder formed by the aggregation of tubular secondary particles, which can be obtained as described above, "tubular" means, for example, a hollow cylindrical shape such as macaroni. The aggregation pattern of the tubular secondary particles is irregular, and no regular pattern is recognizable. The average particle size of primary particles, which form tubes, is normally about 0.02 to 0.1 µm. The average major axis (length of a tube) of tubular secondary particles is normally about 0.5 to 10 µm, and the average minor axis (outer diameter of the cross section of a tube) is normally about 0.1 to 5 µm. The ratio of the average major axis and the minor axis (major axis/minor axis) is normally one or higher, and often it is two or higher. The disintegratable zinc oxide powder of the present invention can be an aggregate of these tubular secondary particles, and the average particle size of the aggregate is normally about 1 to 100 µm. All particle sizes were measured by electron microscope observation.

As described above, the disintegratable zinc oxide powder obtained in the present invention is a relatively large powder. Therefore, the aggregation or dispersal does not take place unlike fine particle powder, and the powder is excellent in usability and handling properties.

In addition, the disintegratable zinc oxide powder of the present invention can provide a high UV protection capability (especially in UV-A protection) and visible-light transmission through its disintegration.

The disintegratable zinc oxide powder of the present invention easily disintegrates to the primary particle level by grinding, friction, etc. Because the zinc oxide disintegrated to the primary particle level is very fine, the transmittance in the UV light region is low and the transmittance in the visible light region is high. As a result, a high UV protection capability and visible-light transmission can be achieved. As described below, for example, in a 5% castor oil dispersion of ground material of the disintegratable zinc oxide powder of the present invention, the transmittance at 360 nm can be made to 20% or lower and further to 15% or lower, and the transmittance at 450 nm can be made to 85% or higher and further to 90% or higher. The disintegratable zinc oxide powder itself cannot provide such an effect because the particle size is too large.

Thus, in order to provide the UV protection effect and transparency, the disintegrated material of the disintegratable zinc oxide powder is necessary to be present on the base material to be protected from UV light at least after the application on the base material.

For example, the disintegrated material ofthe disintegratable zinc oxide powder of the present invention can be present on the skin at least after the cosmetic is applied on the skin. This can be achieved by friction force generated when the cosmetic containing the disintegratable zinc oxide powder is applied on the skin, by grinding the disintegratable zinc oxide powder during the production process of cosmetics, or by blending pre-ground disintegratable zinc oxide powder into the cosmetics. As a result, an excellent UV protection effect and transparency can be provided.

With the use of the disintegratable zinc oxide powder of the present invention, it is possible to produce cosmetics containing the disintegratable zinc oxide powder and/or its disintegrated material.

The cosmetics containing the disintegratable zinc oxide powder can provide an excellent UV protection effect and transparency because the disintegratable zinc oxide powder is easily disintegrated by the friction generated when the cosmetics are applied on the skin.

The disintegratable zinc oxide powder may be disintegrated by being ground during the cosmetics production process. On the other hand, the disintegratable zinc oxide powder may be disintegrated, in advance, by a conventional grinding method before the use in the production of cosmetics.

The disintegratable zinc oxide powder and/or its disintegrated material of the present invention can improve the slip property of cosmetics. Therefore, it can provide cosmetics that are also excellent in feeling in use.

In order to mechanically disintegrate the disintegratable zinc oxide powder, known means can be used. Examples of the means for grinding include a three roll mill, ultrasonic disintegrator, bead mill, motor mill, ring mill, atomizer, and a pulverizer. So far as the objective can be achieved, there is no special limitation to the means.

The blending quantity of the disintegratable zinc oxide powder and/or its disintegrated material in cosmetics are suitably determined depending upon the purpose, the blending quantity is normally 0.001 mass % or higher in cosmetics, and preferably 1 mass % or higher. If the blending quantity is too small, the desired effect may not be achieved. On the other hand, the upper limit is not restricted in particular. Even when a large amount is blended, no excessive whiteness is caused on the skin and the feeling in use is good. However, the blending quantity is normally 50 mass % or lower from the standpoint of mixing with other components, and preferably 30 mass % or lower.

For the disintegratable zinc oxide powder and its disintegrated material ofthe present invention, known surface treatments may be performed as necessary. Examples thereof include treatments with: fatty acid soaps such as aluminum stearate and zinc myristate; waxes such as candelilla wax and carnauba wax; silicones such as methylpolysiloxane and cyclic silicone oils; dextrin fatty acid esters such as dextrin palmitate; and fatty acids such as myristic acid and stearic acid.

In addition to the disintegratable zinc oxide powder and/or its disintegrated material other components normally used in cosmetics can be blended in the cosmetics. Examples include oils, moisturizers, surfactants, pigments, dyes, powders, antioxidants, preservatives, pH adjusters, chelating agents, perfumes, UV absorbers, whitening agents, water, and various drugs.

The cosmetics of the present invention can take any shape such as a powder, solid, paste, liquid, emulsion, or solid-liquid separated state.

Examples of the products include basic cosmetics such as lotions, milky lotions, and creams; makeup cosmetics such as foundations, pre-makeup, lipsticks, eye shadows, cheek colors, eye liners, nail enamels, and mascaras; and hair cosmetics such as hair treatments, hair creams, hair liquids and setting lotions. It is effective, in particular, to blend the disintegratable zinc oxide powder and/or its disintegrated material of the present invention into sunscreen cosmetics for UV light protection.

The disintegratable zinc oxide powder and its disintegrated material of the present invention can be applied to other applications, in which UV light protection is an objective, in addition to cosmetic application. Examples include resin compositions, paints, inks, and coating compositions; however, they are not limited to these applications.

### EXAMPLES

### Test Example 1 Production of disintegratable zinc oxide powder

To 100 mL of 1 mol/L zinc chloride aqueous solution in a flask, 0.1 moles of acetic acid was added and solubilized. To this solution, 0.2 mol/L sodium carbonate aqueous solution was dropwise added at a rate of 1.5 mL/min at 25 °C while the solution was being stirred at 250 rpm with a double-blade stirrer. The pH of the reaction solution was monitored over time, and the addition was continued to pH 8.

Then, the obtained precipitate was filtered, washed, dried (105 °C, 12 hours), and fired (400 °C, 3 hours) to obtain the powder of Test Example 1.

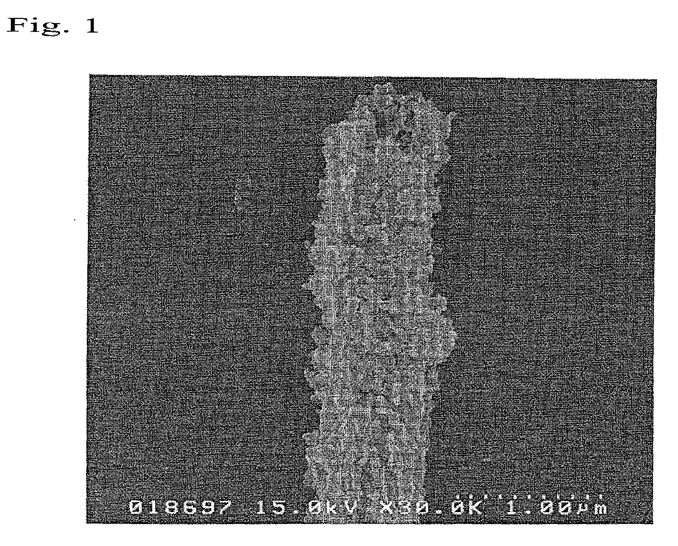

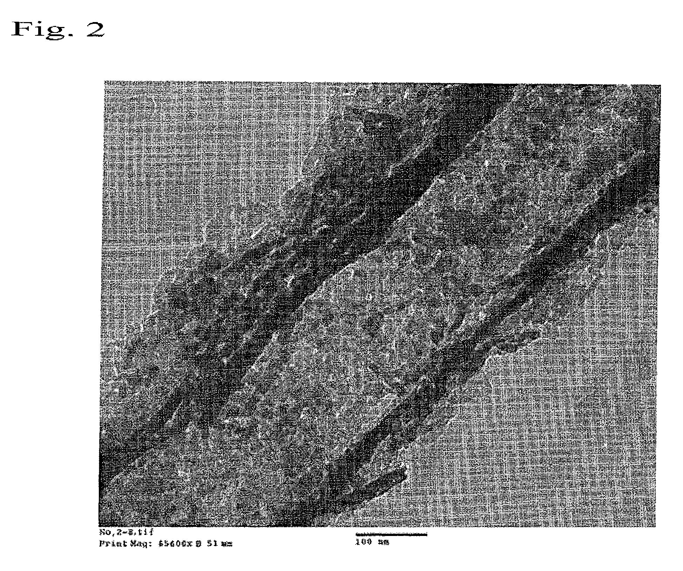

The obtained powder was confirmed to be zinc oxide by the results of X-ray diffraction. This powder was observed with a scanning electron microscope (SEM) and a transmission electron microscope (TEM). As shown in Fig. 1 and Fig. 2, fine primary particles were aggregated, and tubular secondary particles with the major axis of 1 µm or more were formed. The obtained powder was formed by the further aggregation of these tubular secondary particles.

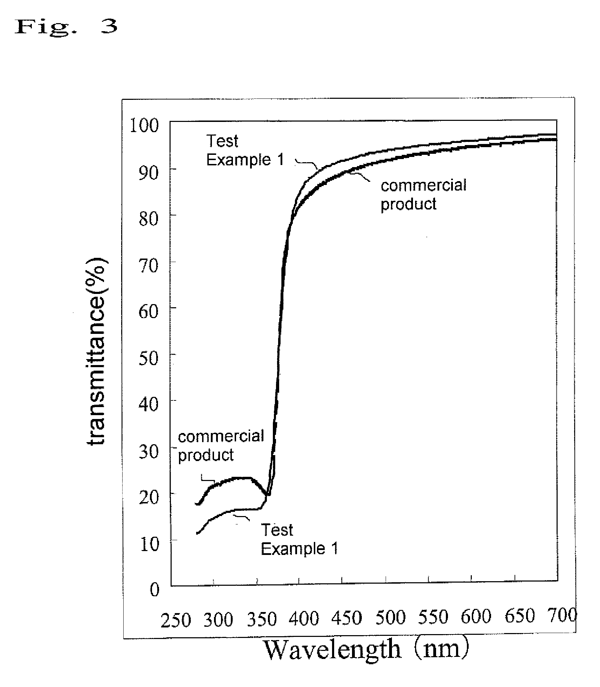

In 3g of castor oil, 2g of zinc oxide powder of Test Example 1 was sufficiently ground and dispersed with a three-roll mill. The obtained dispersion was further diluted with castor oil to a powder concentration of 5 mass %. With an applied film thickness of 5 µm, the transmittance was measured in the range of 280 to 560 nm. For comparison, a castor oil dispersion of commercial ultrafine particulate zinc oxide powder (product of Sakai Chemical Industry Co., Ltd., Finex-50, average particle size: 60 nm) was measured in the same way The results are shown in Fig. 3.

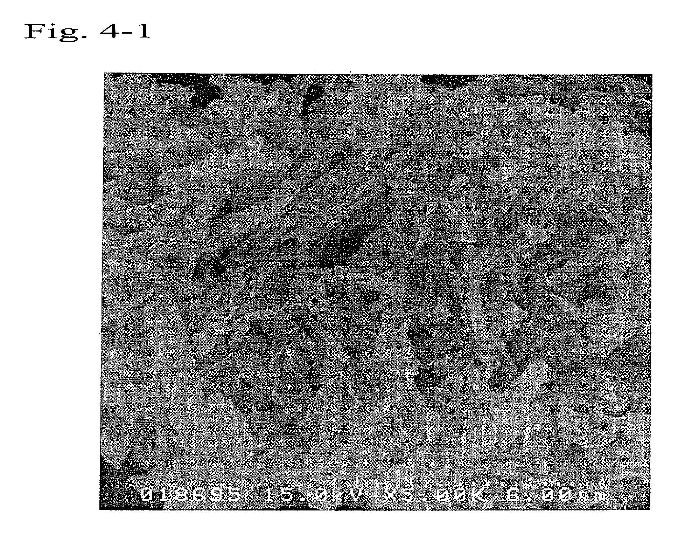

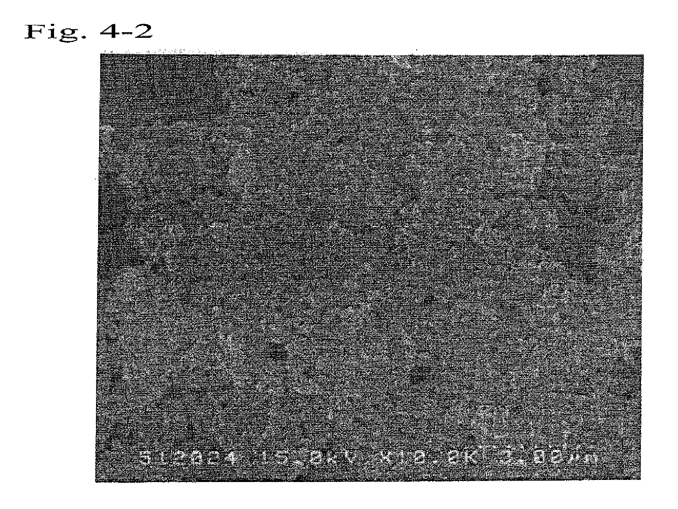

As shown in Fig. 3, Test Example 1 achieved a higher UV protection capability and visible-light transmission than the existing ultrafine particulate zinc oxide powder. Castor oil was removed from the dispersion of Test Example 1 by washing with acetone, and the electron microscope observation was conducted. Tubular secondary particles of the zinc oxide powder were found to be disintegrated to fine primary particles. SEM micrographs before and after dispersion in castor oil are shown in Fig. 4-1 and Fig. 4-2, respectively.

When a mixture of the zinc oxide powder of Test Example 1 and castor oil (without grinding) was extended on the skin with fingers, the zinc oxide powder was similarly disintegrated.

As described above, a UV protection capability and visible-light transmission (transparency) can be achieved by the disintegration of the disintegratable zinc oxide powder of the present invention to the primary particle level by the grinding or by the friction at the time of application.

### Test Example 2 Effect of acid

Powders were obtained in the same way as Test Example 1 by using acids listed in Table 1 instead of acetic acid.

**Table 1:**

| Text Example No. | Acid (Amount) | Shape* |
|---|---|---|
| 2-1 | None (0mol) | ○ |
| 2-2 | Propionic acid (0.1mol) | ○ |
| 2-3 | Maleic anhydride (0.05mol) | ○ |
| 2-4 | Hydrochloric acid (0.1mol) | × |
| 2-5 | Phosphoric acid (0.1mol) | × |

| | | |
|---|---|---|
| * Evaluation criteria: ○: structure in which the secondary particles, which are formed by the aggregation of fine primary particles, are further aggregated ×: significantly sintered powder of primary particles and/or secondary particles | | |

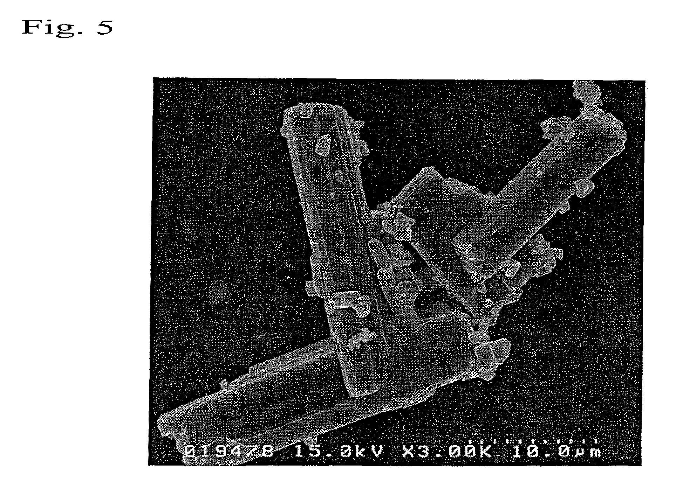

In all cases, powders were confirmed to be zinc oxide based on the results of X-ray diffraction. However, in the cases of mineral acids, such as phosphoric acid and hydrochloric acid (Test Examples 2-4 and 2-5), dense sintered powder was produced and an aggregated structure of the present invention could not be achieved. An SEM micrograph of the obtained powder with the use of phosphoric acid (Test Example 2-5) is shown in Fig. 5.

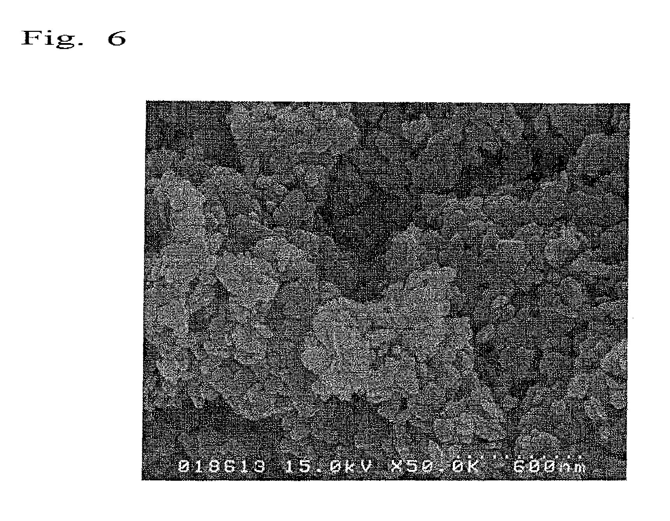

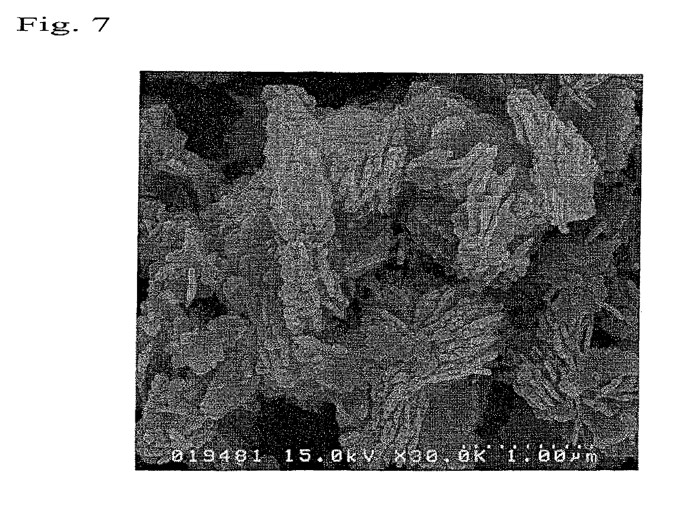

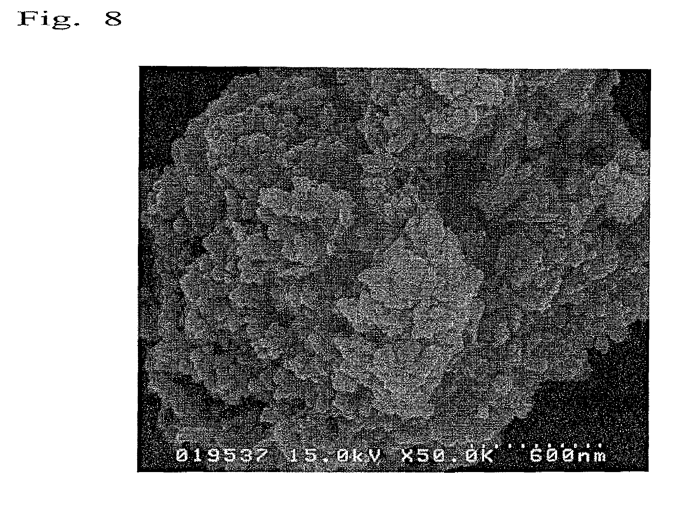

On the other hand, when an acid was not used (Test Example 2-1) or another carboxylic acid was used (Test Example 2-2 or 2-3), it was possible to obtain powder in which rod-like, plate-like, or block-like aggregated secondary particles were formed from fine primary particles instead of tubular aggregated secondary particles, and the secondary particles were further aggregated to form the powder. SEM micrographs for Test Examples 2-1 to 2-3 are shown in Figs. 6 to 8, respectively.

When propionic acid or maleic anhydride was used (Test Example 2-2 or 2-3), the disintegratability and functionality could be achieved though they were inferior than the case in which acetic acid was used (Test Example 1). However, when an acid was not used (Test Example 2-1), the aggregation was dense, the disintegratability was low, and the UV protection capability and the visible-light transmission were very low (refer to Test Example 4).

Thus, a carboxylic acid is presumed to act on the formation of disintegratable zinc oxide powder, and acetic acid is presumed to act specifically on the formation of tubular secondary particles.

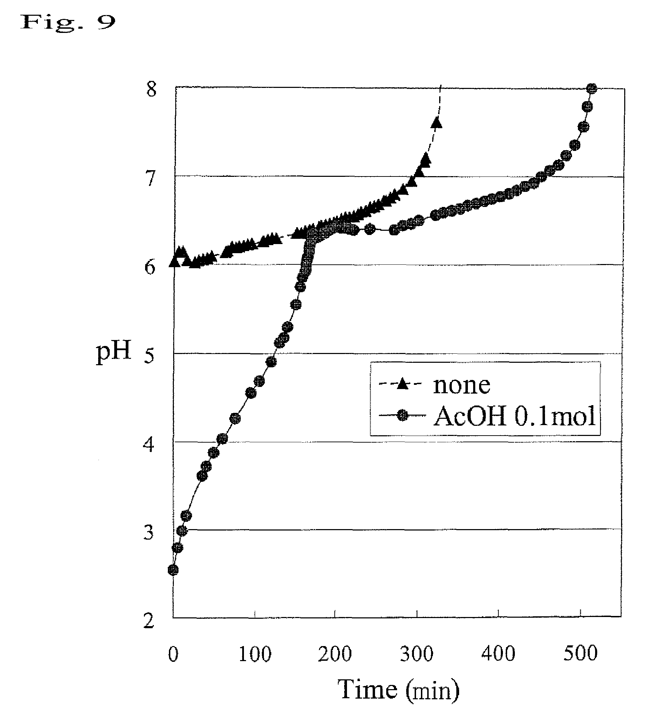

Fig. 9 shows the relationship plots between the pH of reaction solution and the dropping time for Test Example 1 (acetic acid was used) and Test Example 2-1 (acid was not used).

In Test Example 1, where acetic acid was used, the pH of reaction solution almost linearly became higher with an increase of the added amount of sodium carbonate aqueous solution and had an inflection point in the vicinity of pH 4.5. The viscosity of the reaction solution gradually increased and the solution became gelatinous.

In the vicinity ofpH 6.4, a white precipitate started to appear. Once the precipitation started, an increase in pH was very mild with the progress of the addition of sodium carbonate aqueous solution because the carbonate ion is consumed for the precipitation of a zinc salt. When the precipitation is completed, the pH increased drastically

As described above, in Test Example 1, the neutralization reaction proceeded in two steps: the first step up to the precipitation starting point (about pH 6.4) and the following second step.

On the other hand, in Test Example 2-1, where no acid was used, the precipitation started immediately after the start of the addition of sodium carbonate aqueous solution, and the reaction proceeded approximately in one step. As shown in Fig. 6, the obtained zinc oxide powder had a structure where the secondary particles, which were formed by the aggregation of fine primary particles, were further aggregated; however the disintegratability and functionality were unsatisfactory.

In Test Examples 2-2 to 2-5, where various acids were used, the neutralization reaction proceeded in two steps in all cases. As described above, however, the shape of powder, disintegratability, and functionality were different depending upon the types of used acids.

### Test Example 3 Effect of alkaline chemicals

The powder of Test Example 3 was obtained in the same way as the powder of Test Example 1 except for the use of 1 mol/L sodium hydroxide aqueous solution instead of 0.2 mol/L sodium carbonate aqueous solution. The obtained powder was confirmed to be zinc oxide by X-ray diffraction. The powder of Test Example 3 was an aggregate of the secondary particles, which were formed by the dense aggregation of primary particles.

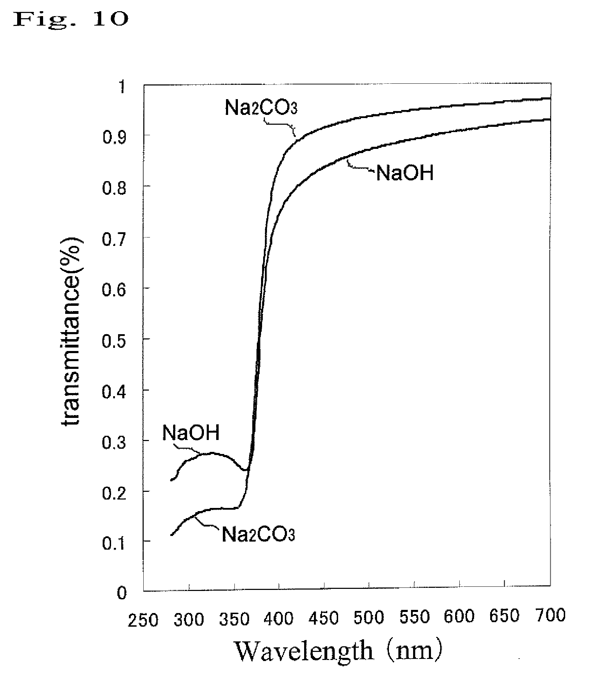

Fig. 10 shows spectral transmittance curves for Test Example 1 and Test Example 3. As seen from Fig. 10, when sodium hydroxide was used as an alkaline chemical (Test Example 3), the UV protection capability and the visible-light transmission were low.

Thus, it is considered that an alkali carbonate is necessary for the formation of disintegratable zinc oxide powder.

### Test Example 4 Concentration of carboxylic acid

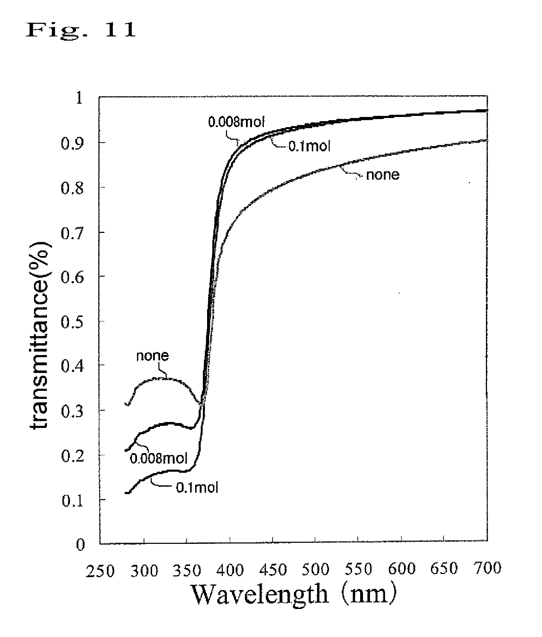

In Test Example 1, the amount of used acetic acid was varied (0 moles, 0.008 moles, or 0.1 moles in 100 mL, in which mole ratio of zinc:acetic acid was 1:0, 1:0.08, or 1:1), and their spectral transmittances for obtained powders were compared. The results are shown in Fig. 11.

As seen from Fig. 11, when acetic acid was not used, both UV protection capability and visible light transmittance were low and unsatisfactory. When the amount of used acetic acid was increased, the UV protection capability and visible light transmittance have improved. On the other hand, the excess use of acetic acid did not significantly improve the effects.

Accordingly, the amount of the carboxyl group of a carboxylic acid is preferably 0.1 to 10 times in moles relative to the amount of zinc, and more preferably 0.5 to 5 times in moles.

A powder was obtained in the same way as the powder of Test Example 1 except for the use of different amounts of acetic acid, and the formation of tubular secondary particles was investigated and the results are shown in Table 2.

As shown in Table 2, all powders were an aggregate of secondary particles, which were formed by the aggregation of fine primary particles. When the amount of used acetic acid was too small, the formation of tubular secondary particles was not recognized. When the amount of used acetic acid was too large, the tubular shape had a tendency to be lost.

Based on these, the amount of acetic acid necessary for the formation of tubular particles is 1 to 10 times in moles relative to the amount of zinc, and preferably 1 to 5 times in moles.

**Table 2:**

| Amount to Zn (times in moles) | Tubular shape* |
|---|---|
| 0 | × |
| 0.2 | × |
| 1.0 | ○ |
| 5.0 | ○ |
| 10.0 | ○~Δ |

| | |
|---|---|
| * Evaluation criteria: ○: tubular Δ: tubular, but the loss or incompletion of tubular shape was observed. ×: not tubular | |

### Test Example 5 Concentration of alkali carbonate

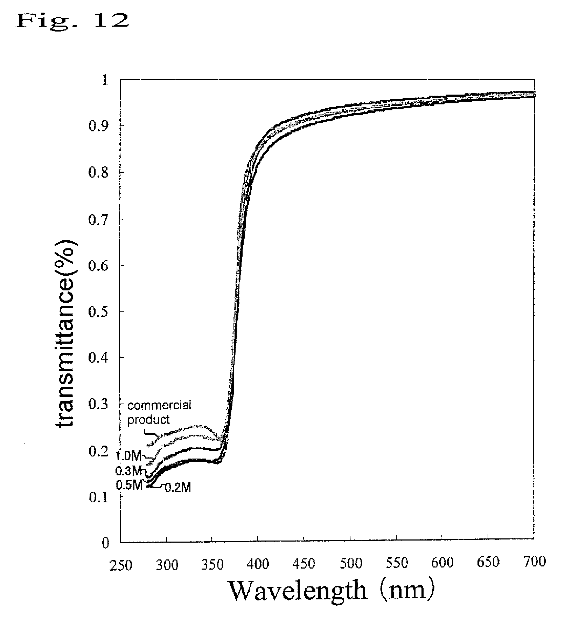

Fig. 12 shows a comparison of spectral transmittances among the powders that were obtained when the concentration of sodium carbonate aqueous solution was varied (0.2, 0.3, 0.5, or 1.0 mol/L) but the addition rate of sodium carbonate aqueous solution was kept at a constant value (1.5 mL/min) in Test Example 1.

As seen from Fig. 12, even when the concentration of sodium carbonate aqueous solution was varied, the high UV protection capability and the visible-light transmission could be achieved. However, when the concentration of sodium carbonate was too high, the UV protection capability and the visible-light transmission had a tendency to decrease. If the concentration is too low, the reaction time becomes significantly long and the reaction is not efficient.

Accordingly, the concentration of alkali carbonate aqueous solution is preferably 0.1 to 10 mol/L, and more preferably 0.3 to 5 mol/L.

A powder was obtained in the same way as the powder of Test Example 1 except for the use of different concentrations of sodium carbonate aqueous solution, and the formation of tubular particles were investigated and the results are shown in Table 3.

**Table 3:**

| Concentration of sodium carbonate aq. sol. (mol/L) | Tubular shape* |
|---|---|
| 0.05 | × |
| 0.2 | ○ |
| 0.5 | ○ |
| 1.0 | ○~Δ |
| 2.0 | Δ |

| | |
|---|---|
| * Dropping rate of sodium carbonate aqueous solution: 1.5 mL/min | |

As shown in Table 3, all powders were an aggregate of secondary particles, which were formed by the aggregation of fine primary particles. When the dropping rate was kept constant and the concentration of sodium carbonate aqueous solution was increased, there was a trend that the tubular shape is lost. On the other hand, when the concentration was too low, tubular particles were not formed. In addition, the reaction time becomes significantly long and the reaction is not efficient.

Accordingly, the concentration of sodium carbonate aqueous solution is preferably 0.1 to 2 mol/L, and more preferably 0.2 to 1.5 mol/L.

### Test Example 6 Dropping rate of alkali carbonate

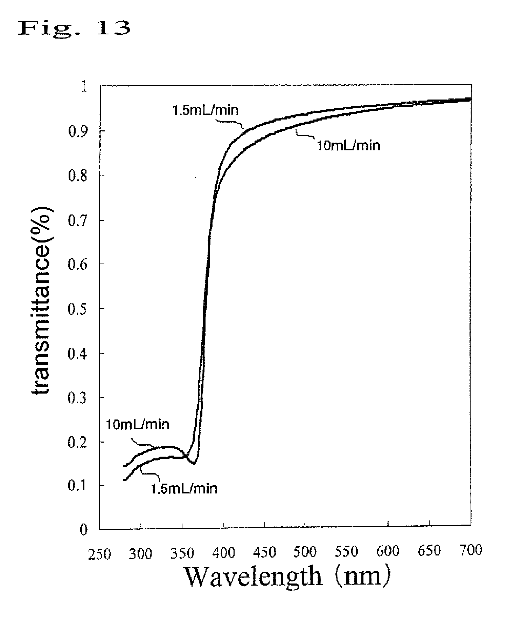

Fig. 13 shows a comparison of spectral transmittances between the powders that were obtained when the dropping rate of sodium carbonate aqueous solution was varied (1.5 or 10.0 mL/min) but the concentration of sodium carbonate aqueous solution was kept at a constant value (0.2 mol/mL) in Test Example 1.

As seen from Fig. 13, when the dropping rate of sodium carbonate was too high, the UV protection capability and the visible-light transmission had a tendency to decrease. If the dropping rate is too low, the reaction time becomes significantly long and the reaction is not efficient.

Accordingly, the dropping rate of sodium carbonate aqueous solution is preferably 0.1 to 10 mL/min per 100 mL of zinc aqueous solution, and more preferably 1 to 5 mL/min.

Powder was prepared in the same way as Test Example 1 except that the dropping rate of sodium carbonate aqueous solution was varied; the formation of tubular particles was investigated and the results are shown in Table 4.

**Table 4:**

| Dropping rate of sodium carbonate aq. sol (mL/min) | Tubular shape* |
|---|---|
| 1.5 | ○ |
| 5.0 | Δ |
| 10.0 | × |

| | |
|---|---|
| * the concentration of sodium carbonate aqueous solution: 0.2 mol/L | |

As shown in Table 4, all powders were further aggregates of the secondary particles formed from fine primary particles. When the dropping rate was increased but the concentration of sodium carbonate aqueous solution was kept constant, tubular secondary particles were not formed. On the other hand, if the dropping rate is too small, the reaction time becomes significantly long and the reaction is not efficient.

Accordingly, in order to form tubular secondary particles, the preferable dropping rate of sodium carbonate aqueous solution is 0.5 to 5 mL/min per 100 mL of zinc aqueous solution.

### Test Example 7 Production of disintegratable zinc oxide powder

A 0.2 mol/L sodium carbonate aqueous solution was dropwise added, at a rate of 1.5 mL/min and at 25 °C, to a 1 mol/L zinc acetate aqueous solution under stirring until pH 8 to produce the powder. The powder was disintegratable zinc oxide powder with high UV protection capability and visible-light transmission. The present zinc oxide powder is an aggregate powder of tubular secondary particles that are formed by the aggregation of primary particles of zinc oxide.

| Blending Example 1 O/W emulsion | |
|---|---|
| (Water phase) | |
| Purified water | to 100 mass % |
| Dipropylene glycol | 6.0 |
| Ethanol | 3.0 |
| Hydroxyethyl cellulose | 0.3 |
| Disintegratable zinc oxide powder | 5.0 |

| (Oil phase) | |
|---|---|
| Octyl paramethoxycinnamate | 6.0 |
| Glyceryl octyl paramethoxycinnamate | 2.0 |
| 4-tert-Butyl-4'-methoxydibenzoylmethane | 2.0 |
| Oxybenzone | 3.0 |
| Oleyl oleate | 5.0 |
| Dimethylpolysiloxane | 3.0 |
| Petrolatum | 0.5 |
| Cetyl alcohol | 1.0 |
| Sorbitan sesquioleate | 0.8 |
| POE(20) oleyl alcohol | 1.2 |
| Antioxidant | Q.S. |
| Antiseptic | Q.S. |
| Perfume | Q.S. |

| Blending Example 2 W/O cream | |
|---|---|
| (Water phase) | |
| Purified water | to 100 mass % |
| 1,3-Butylene glycol | 10.0 |

| (Oil phase) | |
|---|---|
| Disintegratable zinc oxide powder (hydrophobized) | 20.0 |
| Squalane | 20.0 |
| Glyceryl diisostearate | 5.0 |
| Organophilic montmorillonite | 3.0 |
| Antiseptic | Q.S. |
| Perfume | Q.S. |

| Blending Example 3 Sun-protect oil | |
|---|---|
| Disintegratable zinc oxide powder (hydrophobized) | 10.0 mass % |
| Liquid paraffin. | 48.0 |
| Isopropyl myristate | 10.0 |
| Silicone oil | 30.0 |
| Silicone resin | 2.0 |
| Antioxidant | Q.S. |
| Perfume | Q. S. |

| Blending Example 4 Gel | |
|---|---|
| Disintegratable zinc oxide powder | 10.0 mass % |
| Liquid paraffin | 65.0 |
| Olive oil | 20.0 |
| Organophilic montmorillonite | 5.0 |
| Antioxidant | Q.S. |
| Perfume | Q.S. |

| Blending Example 5 Dual foundation | |
|---|---|
| Silicone-treated talc | 19.2 mass % |
| Silicone-treated mica | 40.0 |
| Disintegratable zinc oxide powder (hydrophobized) | 5.0 |
| Silicone-treated titanium dioxide | 15.0 |
| Silicone-treated iron oxide red | 1.0 |
| Silicone-treated iron oxide yellow | 3.0 |
| Silicone-treated iron oxide black | 0.2 |
| Zinc stearate | 0.1 |
| Nylon powder | 2.0 |
| Squalane | 4.0 |
| Solid paraffin | 0.5 |
| Dimethylpolysiloxane | 4.0 |
| Glyceryl triisooctanoate | 5.0 |
| Octyl methoxycinnamate | 1.0 |
| Antiseptic | Q.S. |
| Antioxidant | Q.S. |
| Perfume | Q.S. |

| Blending Example 6 O/W liquid foundation | |
|---|---|
| Talc | 3.0 mass % |
| Disintegratable zinc oxide powder | 15.0 |
| Iron oxide red | 0.5 |
| Iron oxide yellow | 1.4 |
| Iron oxide black | 0.1 |
| Bentonite | 0.5 |
| Sorbitan POE monostearate | 0.9 |
| Triethanolamine | 1.0 |
| Propylene glycol | 10.0 |
| Purified water | to 100 |
| Stearic acid | 2.2 |
| Isohexadecyl alcohol | 7.0 |
| Glyceryl monostearate | 2.0 |
| Liquid lanolin | 2.0 |
| Liquid paraffin | 2.0 |
| Antiseptic | Q. S. |
| Perfume | Q.S. |

| Blending Example 7 W/O foundation (2-phase type) | |
|---|---|
| Hydrophobized talc | 7.0 mass % |
| Disintegratable zinc oxide powder (hydrophobized) | 12.0 |
| Silicic anhydride | 2.0 |
| Nylon powder | 4.0 |
| Color pigment | 2.0 |
| Octamethylcyclotetrasiloxane | 10.0 |
| pentaerythritol rosin acid ester | 1.5 |
| Neopentylglycol diisooctanoate | 5.0 |
| Squalane | 2.5 |
| Glyceryl triisooctanoate | 2.0 |
| Polyoxyethylene dimethylpolysiloxane | 1.5 |
| Purified water | to 100 |
| 1,3-Butylene glycol | 4.0 |
| Ethanol | 7.0 |

| Blending Example8 Powdery foundation | |
|---|---|
| Talc | 20.3 |
| Mica | 30.0 |
| Kaolin | 5.0 |
| Disintegratable zinc oxide powder | 10.0 |
| Titanium dioxide | 5.0 |
| Zinc stearate | 1.0 |
| Iron oxide red | 1.0 |
| Iron oxide yellow | 3.0 |
| Iron oxide black | 0.2 |
| Nylon powder | 10.0 |
| Squalane | 6.0 |
| Lanolin acetate | 1.0 |
| Octyl dodecyl myristate | 2.0 |
| Neopentylglycol diisooctanoate | 2.0 |
| Sorbitan monooleate | 0.5 |
| Antiseptic | Q.S. |
| Perfume | Q.S. |

Cosmetics of the above-described blend examples were prepared according to the conventional methods. There was no problem in the product appearance and stability in all cosmetics. When the cosmetic was applied on the skin, the cosmetic achieved a high UV protection capability (especially in UV-A protection) without causing white masking. In addition, the slip property during application was good compared with the case in which the disintegratable zinc oxide powder was not blended.

## Claims

1. A method for producing a disintegratable zinc oxide powder, comprising:
neutralizing an aqueous solution containing a water-soluble zinc salt and a carboxylic acid or an aqueous solution containing a water-soluble zinc carboxylate with an alkali carbonate to produce precipitate; and
firing the precipitate to obtain the zinc oxide powder,
wherein zinc oxide primary particles are aggregated to form secondary particles, and the secondary particles are further aggregated to form the zinc oxide powder.

2. The method according to claim 1, wherein the water-soluble zinc salt is zinc chloride.

3. The method according to claim 1 or 2, wherein the carboxylic acid is acetic acid.

4. The method according to claim 1, wherein the water-soluble zinc carboxylate is zinc acetate.

5. The method according to any of claims 1 to 4, wherein the alkali carbonate is sodium carbonate.

6. The method according to any of claims 1 to 5, wherein in the aqueous solution containing the water-soluble zinc salt and the carboxylic acid or in the aqueous solution containing the water-soluble zinc carboxylate, the amount of carboxyl group is 0.1 to 10 times in moles relative to the amount of zinc.

7. The method according to any of claims 1 to 6, wherein the alkali carbonate is 0.1 to 10 mol/L aqueous solution thereof per 100 mL of the aqueous solution containing the water-soluble zinc salt and the carboxylic acid or per 100 mL of the aqueous solution containing the water-soluble zinc carboxylate.

8. The method according to claim 7, wherein the alkali carbonate aqueous solution is dropwise added, for neutralization, at a rate of 0.1 to 10 mL/min per 100 mL of the aqueous solution containing the water-soluble zinc salt and the carboxylic acid or per 100 mL ofthe aqueous solution containing the water-soluble zinc carboxylate.

9. The method according to claim 1, wherein an aqueous solution containing zinc chloride and acetic acid or an aqueous solution containing zinc acetate is neutralized with sodium carbonate to produce precipitate, and the precipitate is fired to obtain the zinc oxide powder, wherein zinc oxide primary particles are aggregated to form tubular secondary particles, and the tubular secondary particles are further aggregated to form the zinc oxide powder.

10. The method according to claim 9, wherein the sodium carbonate is 0.1 to 2 mol/L aqueous solution thereof per 100 mL of the aqueous solution containing zinc chloride and acetic acid or per 100 mL of the aqueous solution containing zinc acetate.

11. The method according to claim 9 or 10, wherein in the aqueous solution containing zinc chloride and acetic acid or in the aqueous solution containing zinc acetate, the amount of acetic acid is 1 to 10 times in moles relative to the amount of zinc.

12. The method according to any of claims 9 to 11, wherein the sodium carbonate aqueous solution is dropwise added, for neutralization, at a rate of 0.5 to 5 mL/min per 100 mL of the aqueous solution containing zinc chloride and acetic acid or the aqueous solution containing zinc acetate.

13. A disintegratable zinc oxide powder, wherein zinc oxide primary particles are aggregated to form secondary particles, and the secondary particles are further aggregated to form the zinc oxide powder, which is obtained by the method according to any of claims 1 to 8.

14. The disintegratable zinc oxide powder according to claim 13, wherein the average particle size of the primary particles is 0.02 to 0.1 µm, the average particle size of the secondary particles is 0.5 to 10 µm, and the average particle size of the powder, which is formed by the aggregation of the secondary particles, is 1 to 100 µm.

15. A disintegratable zinc oxide powder, wherein zinc oxide primary particles are aggregated to form tubular secondary particles, and the tubular secondary particles are further aggregated to form the zinc oxide powder.

16. The disintegratable zinc oxide powder according to claim 15, wherein the powder is obtained by the method according to any of claims 9 to 12.

17. The disintegratable zinc oxide powder according to claim 15 or 16, wherein the average major axis of the tube is 0.5 to 10 µm.

18. The disintegratable zinc oxide powder according to any of claims 15 to 17, wherein the ratio of major axis/minor axis of the tube is one or higher.

19. A cosmetic, comprising the disintegratable zinc oxide powder according to any of claims 14 to 16 and/or its disintegrated material.
